# EUROPEAN PATENT APPLICATION

(11) **EP 3 483 606 A1**
(43) Date of publication of application: **15.05.2019**
(21) Application number: 16908199.9
(22) Date of filing: 08.07.2016
(51) Int. Cl.: G01N 33/48, G01N 1/10, G01N 33/493

(54) **HEALTH MONITORING SYSTEM, HEALTH MONITORING METHOD, AND HEALTH MONITORING PROGRAM**

(71) Applicant: Symax Inc., Tokyo 125-0031 (JP)
(72) Inventor: TSURUOKA, Maria, Tokyo 125-0031 (JP); WADA, Yoshitaka, Tokyo 125-0031 (JP)
(74) Representative: Epping - Hermann - Fischer
(86) International application number: PCT/JP2016/070338
(87) International publication number: WO 2018/008155

(57) **Abstract**

A health monitoring system capable of simply analyzing urination information and estimating diseases in monitoring health is provided. A health monitoring system according to the present invention includes a measurement unit that measures fluid information on fluid in reserved water into which urine of a user of a toilet has flowed, an interpretation unit that interprets the urine by analyzing a fluid model obtained by modeling a region in which the fluid flows on the basis of the fluid information, and an inference unit that infers a disease of the user on the basis of urination information of the urine, and the measurement unit includes a cartridge that stores films of which color changes according to components of the reserved water into which the urine has flowed, and extrudes the film to be used for measurement from the cartridge each time the measurement unit measures the fluid information.

## Description

### BACKGROUND OF THE INVENTION

### Field of the Invention

The present invention relates to a health monitoring system, a health monitoring method, and a health monitoring program and, more particularly, to a health monitoring system that is installed in a toilet to interpret urination and estimate a disease, a health monitoring method, and a health monitoring program.

### Description of Related Art

In response to the recent growth in health consciousness, there are a large number of services for analyzing a urinary status (amount or composition thereof), monitoring a state of health, and giving advice in the related art. When there is an abnormality in the body of a person, the urinary status is easily changed, and monitoring the urinary status from day to day in order to detect abnormality in the body may be effective.

As such a urine analysis technique, for example, Japanese Unexamined Patent Application Publication No. 2013-36817 discloses a urination information measurement device that stores data indicating a correlation between a concentration of a specific component in actually measured urine of a person at one time and a concentration of the specific component in the actually measured entire urine of a day, converts to and obtains the concentration of the specific component in the entire urine of a day of a measurement target person using the correlation, and calculates an excretion volume of the specific component in the entire urine of a day of the measurement target person from the obtained concentration.

Further, Japanese Unexamined Patent Application Publication No. 2013-90748 discloses a urination information measurement device that calculates a urination volume and a urine flow rate using a bowl of a toilet in which urine is stored, and a urine data measurement means that measures a volume or weight of the urine stored in the bowl. The urination measurement device described in Japanese Unexamined Patent Application Publication No. 2013-90748 calculates the urination volume and the urine flow rate using respective water levels or a rate of change in water level at the time of the start of urination or at the end of urination and applies a predetermined vibration model to the calculated data to perform a process using a particle filter, thereby calculating urination information.

### SUMMARY OF THE INVENTION

However, in the invention described in Japanese Unexamined Patent Application Publication No. 2013-36817, the invention is mainly constituted of a casing and a sensor unit. The casing is gripped with the hand of a measurement target person or the like, and it is necessary for the urine excreted by the measurement target person to be sprinkled on the sensor unit, the usability thereof may not necessarily be sufficient.

Further, in the invention described in Japanese Unexamined Patent Application Publication No. 2013-90748, as a means that measures a volume or weight of urine stored in the bowl of the toilet, water level data of reserved water inside the bowl is used or sewage pressure measurement of a sewage pipe is used, and elements constituting the toilet are used. Accordingly, the invention cannot be applied to existing toilets. Therefore, in the urination information measurement device described in Japanese Unexamined Patent Application Publication No. 2013-90748, versatility is poor and usability is not necessarily sufficient.

Therefore, an object of the present invention is to provide a health monitoring system that is simple and convenient for urination interpretation, such as urine component analysis, and inference of diseases based on interpretation results, a health monitoring method, and a health monitoring program.

A health monitoring system according to the present invention includes a measurement unit that measures fluid information on fluid in reserved water into which urine of a user of a toilet has flowed; an interpretation unit that interprets the urine by analyzing a fluid model obtained by modeling a region in which the fluid flows on the basis of the fluid information; and an inference unit that infers a disease of the user on the basis of urination information of the urine, wherein the measurement unit includes a cartridge that stores films of which color changes according to components of the reserved water into which the urine has flowed, and extrudes the film to be used for measurement from the cartridge each time the measurement unit measures the fluid information.

Further, in the health monitoring system according to the present invention, the measurement unit may further include an extrusion mechanism that extrudes the film used for the measurement among a plurality of films stacked and stored in the cartridge to the outside of the cartridge.

Further, in the health monitoring system according to the present invention, the measurement unit may further include an arm including a clamping portion that clamps the film extruded from the cartridge, the measurement unit operating the arm such that the film clamped by the clamping portion is immersed in the reserved water into which the urine of the user has flowed.

Further, in the health monitoring system according to the present invention, the measurement unit may further include a rotation mechanism rotatably connected to one end of the arm, and the arm may be rotated by the rotation mechanism to move the clamping portion provided at the other end of the arm to a position at which the clamping portion is immersed in the reserved water into which the urine of the user has flowed.

Further, the health monitoring system according to the present invention may include an imaging unit including an imaging means that images film to generate imaging information, wherein the measurement unit may move film immersed in the reserved water into which the urine of the user has flowed, to a position at which the imaging unit can image the film by moving the arm.

Further, in the health monitoring system according to the present invention, the cartridge may be detachable from the measurement unit, and in the measurement unit, when the film stored in the cartridge runs out, the cartridge may be able to be replaced with a new cartridge in which a plurality of films are stored.

Further, the health monitoring system according to the present invention may include a storage unit that stores shape information of a bowl of each toilet and water volume information of the reserved water, wherein the fluid information may include water temperature information generated by measuring a water temperature of at least one of the reserved water or the reserved water into which the urine has flowed, and the interpretation unit may interpret the urine on the basis of at least one of the shape information and the water volume information, in addition to the fluid information.

Further, the health monitoring system according to the present invention may measure a potential difference between two electrodes immersed in the reserved water or reserved water into which the urine has flowed to generate voltage information, and the health monitoring system may include a correction unit that corrects the voltage information on the basis of the water volume information and the urination information including a urine volume of the urination; and an analysis unit that analyzes urine components on the basis of the corrected voltage information.

Further, in the health monitoring system according to the present invention, when at least one of the water temperature information or the voltage information reaches a predetermined threshold value, the measurement unit may start or end measurement of at least one of a water temperature and an electrical potential difference of the reserved water into which the urine has flowed.

Further, in the health monitoring system according to the present invention, the inference unit may create a feature vector from the imaging information, identify the created feature vector using training data, and estimate a disease on the basis of the identified feature vector.

Further, the health monitoring system according to the present invention may further include a user identification unit that identifies a user on the basis of user identification information output from a terminal or an IC card possessed by the user, wherein the inference unit may estimate a disease of each of the users on the basis of results of the identification.

Further, in the health monitoring system according to the present invention, the user identification unit may further include a measurement unit that measures a weight of the user received by a toilet seat of the toilet when the user uses the toilet seat to generate weight information, the user identification unit identifying the user on the basis of the weight information.

A health monitoring method according to the present invention includes a measurement step of measuring fluid information on fluid in reserved water into which urine of a user of a toilet has flowed; an interpretation step of interpreting the urination by analyzing a fluid model obtained by modeling a region in which the fluid flows, on the basis of the fluid information; an inference step of estimating a disease of the user on the basis of the urination information of the urine; and an extrusion step of extruding a film used for measurement from a cartridge that stores a film of which color changes according to components of reserved water into which the urine has flowed each time the fluid information is measured.

A health monitoring program according to the present invention is a health monitoring program for controlling a computer, the health monitoring program causing the computer to realize: a measurement function of measuring fluid information on fluid in reserved water into which urine of a user of a toilet has flowed; an interpretation function of interpreting the urination by analyzing a fluid model obtained by modeling a region in which the fluid flows, on the basis of the fluid information; an inference function of estimating a disease of the user on the basis of the urination information of the urine; and an extrusion function of extruding a film used for measurement from a cartridge that stores a film of which color changes according to components of reserved water into which the urine has flowed each time the fluid information is measured.

The health monitoring system according to the present invention includes the measurement unit that measures fluid information on fluid in reserved water into which urine of a user of a toilet has flowed; the interpretation unit that interprets the urine by analyzing a fluid model obtained by modeling a region in which the fluid flows on the basis of the fluid information; and the inference unit that infers a disease of the user on the basis of urination information of the urine.

With this configuration, since a measurement target person can measure urine components simply when excreting urine as usual by installing the health monitoring system according to the present invention in an existing toilet in measurement of the urine components, the measurement target person can more simply and hygienically measure urine components than measurement performed by sprinkling urine on a device, and usability can be improved.

Further, since the health monitoring system and the health monitoring method according to the present invention interprets a movement of a fluid through fluid simulation and analyze the urine volume, it is possible to take into account how much the urine is diluted by the reserved water, and perform accurate analysis.

The health monitoring system and health monitoring method according to the present invention can improve simplicity and convenience in interpretation of urination information and inference of disease.

### BRIEF DESCRIPTION OF THE DRAWINGS

FIG. 1 is a system diagram illustrating an example of a configuration of a health monitoring system 500.
FIG. 2 is a block diagram illustrating a configuration example of the health monitoring system 500.
FIG. 3 is a block diagram illustrating another configuration example of the health monitoring system 500.
FIG. 4 is a diagram schematically illustrating an example of an overview of a health monitoring system 500.
FIG. 5 is a diagram schematically illustrating an example of an overview of a measurement device 200 of the health monitoring system 500.
FIG. 6 is a diagram schematically illustrating an example of an internal structure of a measurement unit 210 constituting the measurement device 200.
FIG. 7 is a diagram schematically illustrating another example of the internal structure of the measurement unit 210 constituting the measurement device 200.
FIG. 8 is a diagram schematically illustrating a configuration example of the measurement unit 210 constituting the measurement device 200.
FIG. 9 is a diagram schematically illustrating a state in which a clamping portion 41 of an arm 40 moves in a vertical direction.
FIG. 10 is a diagram schematically illustrating an example of a structure of a film 90 and a reagent constituting the imaging unit 212.
FIG. 11 is a data conceptual diagram illustrating a data configuration example of a database of data stored in the health monitoring system 500.
FIG. 12 is a data conceptual diagram illustrating an example of a data configuration of association between measurement and analysis results and information such as diseases in the health monitoring system 500.
FIG. 13 is a flowchart showing an example of a process that is executed by the health monitoring system 500.
FIG. 14 is a diagram schematically illustrating an example of an overview of the measurement unit 210.
FIG. 15 is a diagram schematically illustrating another example of an overview of the measurement unit 210.
FIG. 16 is a diagram schematically illustrating a configuration example of the measurement unit 210 constituting the measurement device 200.
FIG. 17 is a schematic diagram illustrating a usage aspect of the measurement unit 210.
FIG. 18 is a diagram illustrating a configuration example of a toilet to which a pressure sensor 800 is attached.

### DETAILED DESCRIPTION OF THE INVENTION

Hereinafter, embodiments of the present invention will be described with reference to the drawings.

### <Embodiment 1>

### <Overview>

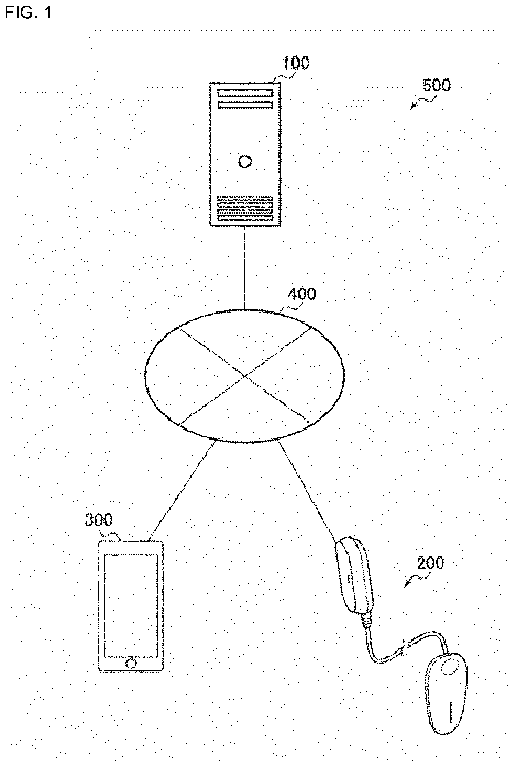

FIG. 1 is a system diagram illustrating an example of a configuration of a health monitoring system according to Embodiment 1 of the present invention.

As illustrated in FIG. 1, the system includes a server 100, a measurement device 200, and a user terminal 300. The server 100 is connected to the measurement device 200 and the user terminal 300 via a network 400. In FIG. 1, only one server 100, one measurement device 200, and one user terminal 300 are illustrated for simplicity of description, but it is obvious that there may be more than one server 100, more than one measurement device 200, and more than one user terminal 300. A specific device of the user terminal 300 is not limited to a smartphone as illustrated in FIG. 1, and may be, for example, a mobile terminal, a tablet terminal, a personal computer, or another electronic device. Examples of the user terminal 300 include a computer (for example, a desktop computer, a laptop, or a tablet), a media computer platform (for example, a cable, a satellite set-top box, or a digital video recorder), a handheld computing device (for example, a personal digital assistant (PDA) or an e-mail client), a mobile phone (for example, a smartphone or a feature phone), a wearable terminal (for example, a glasses type device or a clock type device), another type of computer, or a communication platform, but the present invention is limited thereto. Further, the system may use a cloud service (including both a public cloud and a private cloud), or may provide a service by physically providing a shared or dedicated server within a target facility.

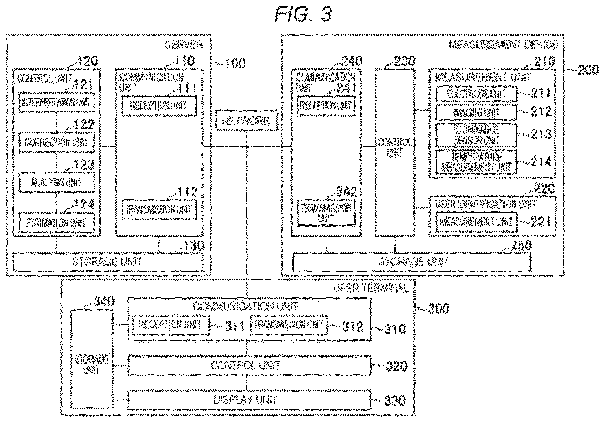

The user terminal 300 has an application (hereinafter referred to as a "health monitoring application") for displaying state of health monitoring results (including analysis results and an inference results) which is a part of the health monitoring system according to Embodiment 1 of the present invention mounted thereon, and a state of health of a user can be checked by browsing the display of the health monitoring application, as illustrated in FIG. 3.

For example, as illustrated in FIG. 3, in the health monitoring system 500, the measurement device 200 is installed in an existing toilet or the like, the measurement device 200 measures the fluid information on the fluid in the reserved water into which the urine of the user of the toilet has flowed, and the server 100 interpret the fluid model obtained by modeling a region in which the fluid flows, on the basis of the measured fluid information to interpret the urination, such that a disease of the user can be inferred on the basis of the urination information of the interpreted urination. Accordingly, for example, the health monitoring system 500 can determine a sign of a disease or a disease by the user merely performing a usual urination action when staying at home or in a work place, such that a health monitoring service that is simple and convenient and of which sustainability is high can be provided.

Further, the health monitoring system 500 is not limited to being applied to a house or a workplace. The health monitoring system 500 can also be used for patient health management in a nursing care facility or a hospital, and reduce risk on the administrative side. Here, the "urination information" refers to various types of information on urination of a user, and may include urine volume of the urination, urine temperature, urine components, and the like.

It should be noted that the example in which the cloud computing format has been used is shown in Embodiment 1, the present invention is not limited to this example. For example, the health monitoring system 500 may be configured of the measurement device 200 alone, or the measurement device 200 and the user terminal 300. Further, in this example, a service using a cloud service has been shown, but the present invention can also be used for a cloud doctor service (for example, a service for monitoring a state of health or a physical state of a patient over a network) using artificial intelligence (for example, artificial intelligence using machine learning based on deep learning or the like) or a cloud mother service (for example, a service for monitoring a state of health or a physical state of a child over a network).

### <Configuration>

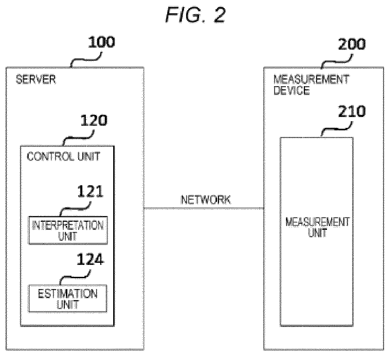

FIG. 2 is a block diagram illustrating a configuration example of the health monitoring system according to Embodiment 1 of the present invention.

As illustrated in FIG. 2, the health monitoring system according to Embodiment 1 includes the measurement device 200 including a measurement unit 210, and a server 100 including a control unit including an interpretation unit 121 and an inference unit 124.

The measurement unit 210 of the measurement device 200 measures fluid information on fluid in reserved water into which the urination of the user of the toilet has flowed.

The interpretation unit 121 of the server 100 interprets the urination by analyzing a fluid model obtained by modeling the fluid on the basis of the fluid information measured by the measurement unit 210. Further, the inference unit 124 of the server 100 infers a disease of the user on the basis of the urination information of the urine.

In Embodiment 1 of the present invention, the measurement unit 210 includes a cartridge that stores a film of which color changes according to the components of the reserved water into which the urine has flowed, and extrudes the film to be used for measurement from the cartridge each time the measurement unit measures the fluid information.

As described above, the measurement unit 210 includes the cartridge storing the films that are used for measurement of the fluid information, and the film that is used for measurement is extruded from the cartridge each time the fluid information is measured. Therefore, urine components can be measured without the measurement target person performing work such as preparation of a film that is used for examination. Therefore, the measurement target person can more easily and sanitarily perform measurement than, for example, in measurement that is performed by sprinkling urine on a device, and it is possible to improve usability.

Hereinafter, configurations of the server 100, the measurement device 200, and the user terminal 300 will be described in detail. FIG. 3 is a block diagram illustrating an example of the functional configuration of the server 100, the measurement device 200, and the user terminal 300. It should be noted that a disposition of respective units may be appropriately changed between the server 100, the measurement device 200, and the user terminal 300 according to, for example, an operation environment and situation of each device. For example, the interpretation unit 121, the correction unit 122, the analysis unit 123, and the inference unit 124 of the server 100 may be disposed in the control unit 230 of the measurement device 200 or may be disposed in the control unit 320 of the user terminal 300. As illustrated in FIG. 3, the server 100 includes a communication unit 110, a control unit 120, and a storage unit 130.

Further, the server 100 may have a multistage configuration, and may include, for example, a server (a relay server) installed within a facility, and a server including a specific area or all areas including a plurality of facilities. A transmission timing of the relay server may be, for example, (1) periodically (for example, at regular time intervals determined in consideration of, for example, a capacity of the storage unit 130), and (3) when a storage capacity of the storage unit 250 has reached a threshold value, which is set for the storage capacity of the storage unit 250.

The communication unit 110 includes a reception unit 111 and a transmission unit 112 and has a function of executing communication with the measurement device 200 and the user terminal 300 via the network 400. The communication may be either wired or wireless, and any communication protocol may be used as long as mutual communication can be executed. The network 400 may be, for example, a network such as LTE (Long Term Evolution), LANs (Local Area Networks), WANs (Wide Area Networks), MANs (Metropolitan Area Networks), or ISDNs (Integrated Service Digital Networks), wireless LANs, CDMA (Code Division Multiple Access), Bluetooth (registered trademark), or satellite communication, and may be any network as long as the information processing device 100 or the information processing terminal 300 can communicate with each other regardless of wired or wireless communication.

The reception unit 111 has a function of receiving measurement data or the like from each measurement device 200 and each user terminal 300 via the network 400 and transferring the measurement data to the control unit 120 under the control of the control unit 120. Specifically, the reception unit 111 receives water temperature information of the reserved water inside a bowl of the toilet and water containing the urination of the user of the toilet in the reserved water (hereinafter referred to as "urination-containing water"), voltage information according to a potential difference between electrodes obtained by immersing the electrodes in the urination-containing water, user identification information for identifying the user, illuminance information, and imaging information obtained by imaging the film 90 to which a reagent has reacted in the imaging unit 212 (hereinafter referred to as "imaging information) from the measurement device 200, and transfers the information to the control unit 120.

The transmission unit 112 has a function of transmitting control data or the like to each measurement device 200 and monitoring results data or the like to each user terminal 300 via the network 400 under the control of the control unit 120. Specifically, the transmission unit 112 transmits user information (for example, ID information) stored in the storage unit 130 for control of the user identification unit 220 and, for example, dynamic parameter data necessary for measurement and imaging of the measurement unit 210 and identification of the user identification unit 220 to the measurement device 200, and also transmits display data indicating monitoring results such as the analysis results related to the analyzed urine component and positive and negative inference results related to the inferred disease to the user terminal 300.

The control unit 120 is a processor that includes the interpretation unit 121, the correction unit 122, the analysis unit 123, and the inference unit 124 and has a function of controlling each unit of the server 100. Further, when the analysis result is transferred from the analysis unit 123 and when an inference result is transferred from the inference unit 124, the control unit 120 generates display data for displaying the results on the display unit 330 of the user terminal 300 as text, a table, or a graph. In order to transmit the generated display data to the user terminal 300, the control unit 120 transfers the generated display data to the transmission unit 112.

The interpretation unit 121 has a function of interpreting the urination by interpreting the fluid model obtained by modeling the region in which a fluid flows on the basis of the fluid information. Here, the "fluid information" refers to information necessary for fluid interpretation and includes, for example, shape information of the bowl of the toilet (hereinafter referred to as "shape information"), and water volume information and water temperature information of reserved water inside the bowl of the toilet.

Specifically, the interpretation unit 121 interprets, for example, the urination by interpreting the fluid around the measurement unit 210 and calculating the urine volume on the basis of the fluid model obtained by modeling the fluid flowing around the measurement unit 210 on the basis of at least one of the shape information of the bowl of the toilet, the water volume information or the water temperature information of the reserved water inside the bowl of the toilet, and the like. Further, the interpretation unit 121 may add at least one of information on a toilet environment such as information on the amount of a detergent or the like and information on components of the detergent or the like, in addition to the shape information of the bowl of the toilet, the water volume information and the water temperature information of the reserved water inside the bowl of the toilet, and interpret the urination information, for example, by modeling the fluid on the basis of this information. Accordingly, it is possible to provide a health monitoring system in which it is only necessary to collect only urine and measure the urine volume or to measure the urine volume from a rate of change in water level using a measurement device or the like attached to the bowl of the toilet or a drain pipe, and which is convenient for users.

For modeling of the fluid, for example, it is conceivable to construct a prediction model as to how the water temperature of the reserved water and the urine-containing water will change and the water temperature will ultimately converge on the basis of the water temperature information generated from the measured water temperature of the reserved water and the urine-containing water using regression analysis based on a support vector machine (SVM) or the like, and perform analysis. Further, in the regression analysis, a data structure derived using a kernel method may be combined with the SVM and the analysis may be performed. Further, as another example, it is conceivable to construct a regression model using regression analysis based on a Markov Chain Monte Carlo (MCMC) method and perform analysis. In addition, it is also conceivable to use a finite element method or a computational fluid dynamics (CFD) method as an example of modeling a fluid region using fluid simulation.

The correction unit 122 has a function of correcting the voltage information on the basis of the urination information including the water volume information and the urine volume. Specifically, for example, the correction unit 122 calculates a degree of dilution by dividing the urine volume by a sum of the water volume and the urine volume, and corrects the voltage information from the degree of dilution. Accordingly, it is possible to acquire voltage information considering the dilution due to the reserved water or the like inside the bowl of the toilet, and to determine urine components.

The correction unit 122 has a function of correcting imaging information on the basis of illuminance information. Here, the "illuminance information" is information indicating the illuminance (brightness) (lx) of a film surface of the imaging unit 212. Specifically, for example, the correction unit 122 corrects correcting imaging information by adjusting brightnesses of RGB values to appropriate values on the basis of the illuminance information. Accordingly, it is possible to obtain RGB values considering an influence of an illumination, and accurately perform color measurement.

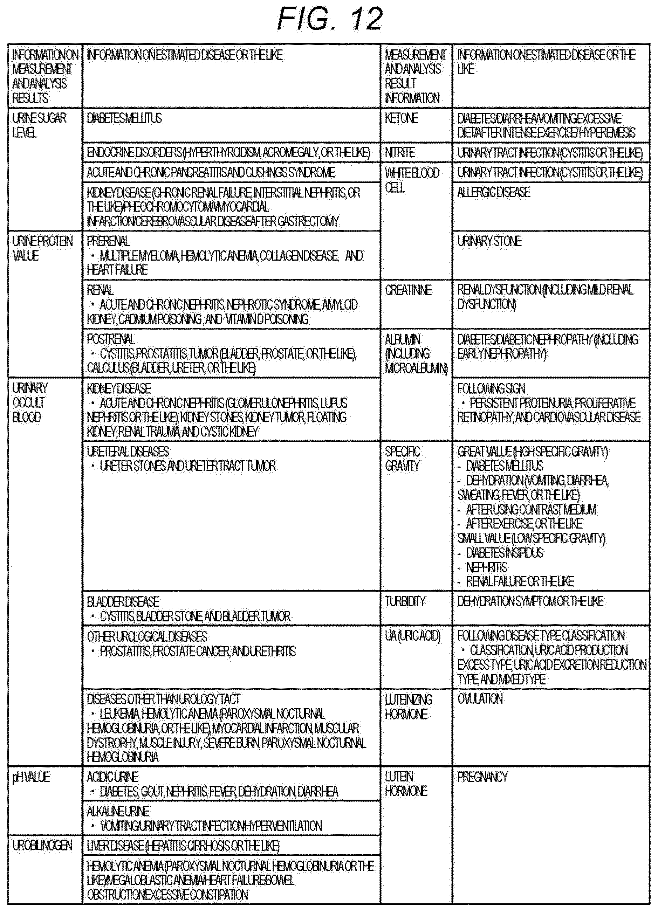

The analysis unit 123 has a function of analyzing the urine components on the basis of the voltage information or the corrected voltage information (hereinafter referred to as "voltage information (after correction)"). The analysis unit 123 specifically analyzes, for example, a molecular concentration of components such as chloride, glucose, potassium, sodium, and urea in the urine on the basis of the voltage information (after correction). Further, it is also possible to analyze a pH value, as illustrated in FIG. 12. Accordingly, even when the urine is diluted with the reserved water, it is possible to perform accurate analysis. Further, the analysis unit 123 transfers analysis results to the control unit 120 in order to generate display data to be displayed on the user terminal 300.

The analysis unit 123 also has a function of analyzing the urine components on the basis of the imaging information or the corrected imaging information (hereinafter referred to as "imaging information (after correction)"). Specifically, for example, the analysis unit 123 measures color of a coloring reaction of a specific component in the urine to a reagent on the basis of the imaging information (RGB value), and analyzes a specific component in the urine corresponding to the color or a concentration thereof. Further, the analysis unit 123 transfers an analysis result to the control unit 120 in order to generate display data to be displayed on the user terminal 300. Accordingly, it is possible to automatically and simply realize the analysis of the specific component in the urine and the concentration thereof using a bioassay (such as immunochromatography) without using, for example, visual observation of a person and without human intervention.

The inference unit 124 has a function of estimating a disease of the user on the basis of the interpreted urination information of the urination. Specifically, for example, the inference unit 124 infers the disease of the user on the basis of the analyzed specific component in the urine (specifically, for example, the concentration of the component). As an example, as illustrated in FIG. 12, a urine sugar value is calculated by analyzing a concentration of glucose in the urine to estimate whether the diabetes is positive or negative. Further, FIG. 12 illustrates an example of association between measurement of other measurement units 210 or analysis results of the analysis unit 123 (referred to as "measurement and analysis results") and information such as disease inferred from the measurement and analysis results. The inference of the inference unit 124 may include inference represented in the example of the association. Further, the inference unit 124 transfers an inference result to the control unit 120 to generate display data to be displayed on the user terminal 300.

In the inference of the inference unit 124, (1) inference using a threshold value and (2) inference using machine learning can be used. For example, in the inference (1), the inference unit 124 compares the measurement result with the threshold value stored in the storage unit 130 and infers the disease, for example, by determining normal (or negative) when the measurement result is within the threshold value and abnormal (or positive) when the measurement result exceeds the threshold value. In the inference (2), the inference unit 124 extracts a feature quantity of the measurement result, and creates a feature vector on the basis of the feature quantity. The created feature vector is identified with reference to dictionary data (data created by using a plurality of cases of a set of pieces of a measurement value and an examination result associated with the measurement value (a result of, for example, whether a disease is positive or negative based on the analysis result and the inference result), training data (teacher data) in machine learning), and the disease is inferred on the basis of a result of the identification. It should be noted that a neutral network (perceptron), an SVM, or the like may be used as a machine learning scheme. Accordingly, inference accuracy of the inference unit 124 can be improved due to a learning effect of the machine learning.

The storage unit 130 has a function of storing various programs, data, and parameters necessary for an operation of the server 100. Specifically, for example, the storage unit 130 stores the fluid information (the shape information of the bowl of the toilet, the water volume information of the reserved water of the toilet), the imaging information, the weight information, the illuminance information, the user identification information, and parameters necessary for an operation of the communication unit 110, the control unit 120, and the storage unit 130. As an example, the storage unit 130 stores information necessary for interpretation, analysis, or the like, the measurement results, and examination results (analysis results and inference results) in various databases (hereinafter referred to as "DBs"), as illustrated in FIG. 12.

Further, the storage unit 130 may store a questionnaire results answered by the user. The health monitoring system according to the present invention collects a questionnaire results on the state of health of the user and uses the questionnaire results for management of the state of health of the user. The questionnaire on the state of health includes, for example, (1) physical symptoms of the user, (2) a mental state of the user, (3) an activity situation of the user, (4) a situation regarding functions of the user, and (5) other information.

For example, (1) the physical symptoms of the user include information on a body of the user and include, for example, a height or weight of the user, blood pressure, and a heart rate at the time of questionnaire. The user may answer items that can be measured by the user at the time of the questionnaire. Further, (1) the physical symptoms of the user include symptoms of the body perceived by the user himself or herself and may include symptoms that the user felt different from usual, such as "feel nauseated", "there is pain", "no power on a body", "tinnitus", "sense of hands and feet paralyzed", and "an entire body is swollen" that are answered using a selection scheme or a description scheme at the time of questionnaire. (1) The physical symptoms of the user are not limited to these examples, and may be any information as long as the information is information on the body of the user. Further, when the items are answered using the selection scheme, the user may select not only YES or NO. For example, the selection may be in the form of selecting numerals. The selection may be in the form of selecting a corresponding one from (0) "absolutely not", (1) "very slightly", (2) "somewhat", (3) "quite", and (4) "very well". It should be noted that in the following questionnaire items, a form for selecting such numbers can be adopted.

Further, (2) a mental state of the user includes, for example, information on a mental state perceived by the user. For example, various items regarding the mental state such as "satisfied", "feel fun", "feel sad", "nervous", "feel uneasy", "not feel motivated" are answered using a selection scheme or a description scheme. Further, (2) the mental state of the user may include information on a relationship between the user and surrounding human beings. For example, items such as "feel close to friends", "feel intimate from family", and "feel close to a boss and colleagues" are answered using a selection scheme or a description scheme. It should be noted that (2) the mental state of the user is not limited to these examples, and may be any information.

Further, (3) the activity situation of the user includes information on a daily activity of the user. For example, items such as "can do work", "can enjoy life", "can sleep well", and "enjoy entertainment" are answered using a selection scheme or a description scheme. Further, (3) the activity situation of the user may include, for example, an activity situation regarding exercises such as sports which the user has performed within a predetermined period of time (for example, within one week). A jogging time, a running time, another time in which sports have been performed, and the like are answered using a selection scheme or a description scheme. It should be noted that (3) the activity situation of the user is not limited to these examples, and may be any information.

Further, (4) The situation regarding the function of the user includes information on a state (function) of a body of the user. For example, items such as "difficult to sleep", "difficult to press a keyboard", "difficult to perform manual work", and "difficult to write characters" are answered using a selection scheme or a description scheme. It should be noted that (4) the function of the user is not limited to these examples, and may be any information.

Further, (5) other information includes, for example, information that the user feels worried. For example, items such as "an ear has become difficult to hear", "an entire body is weak", "feel short of breath", "bothered by hair loss", and "concerned about public eye" are answered using a selection scheme or a description scheme. It should be noted that (5) the other information is not limited to these examples, and may be any information.

Further, for example, a patient who current goes to a hospital may be caused to answer questions about more detailed content and items than in the above questionnaire. In the questionnaire for a patient, for example, a specific organ such as a stomach, an intestine, or head may be designated and the patient may be caused to answer questions about a pain or state of the organ. Further, in the questionnaire for a patient, the patient may be caused to answer questions about medicine being taken.

As described above, the health monitoring system of the present invention collects questionnaires about health from users. The questionnaire about the health of the user is periodically performed, for example, every week and stored in association with a date and time of the questionnaire. Further, the questionnaire about the health of the user is not only periodically performed but may be performed irregularly, for example. Further, the questionnaire regarding the health of the user may be distributed to the user when the results of the analysis from the urination information of the user becomes a specific measurement result. For example, when the uric acid value is higher than a predetermined threshold value (a specific measurement result) in the results of the analysis from the urination information, a questionnaire about health may be performed with respect to the user in order to confirm a meal or drinking situation or the like. Further, for example, when the content of the sugar is higher than a predetermined threshold value (a specific measurement result) in the results of the analysis from the urination information, a questionnaire about health may be performed with respect to the user to confirm an activity situation (for example, exercise situation), or the like of the user. Thus, the questionnaire about the health of the user may be performed at any timing.

Thus, the health monitoring system can store the urination information interpreted from the urination of the user and the questionnaire result in association with each other, thereby providing detailed analysis results and health information to the user. For example, it is possible to discover and infer a sign of the disease of the user by matching the analysis result obtained by performing analysis from the urination information which is a daily measurement result with the questionnaire result. Further, a cause of a subjective symptom of the user analyzed from the questionnaire results can be specified using the results of the analysis from the urination information. Thus, it is possible to perform a detailed study on the basis of the questionnaire results of the user, the daily measurement result, and the analysis result, and to discover a disease of the user at an early stage.

Further, the storage unit 130 may store health checkup results of the user for each user. Since health checkup is usually performed only once a year, the user is likely to lose the health checkup results printed on paper, for example. Further, the health checkup results also in77clude items indicated by numerical values, and the user is likely to forget the items immediately. Therefore, the health monitoring system of the present invention has a function of receiving a notification of the health checkup results of the user from the user, a hospital in which health checkup has been performed, or the like, and uploading the results to the server 100.

The server 100 stores the notified health checkup results in the storage unit 130. Therefore, when there is a request for health checkup results from the user, the server 100 can notify the user of the health checkup results stored in the storage unit 130.

Further, the storage unit 130 may store the health checkup results in association with the measurement results or the examination results of the user for each user. It is possible to confirm an improvement situation or a deterioration situation of the disease found in the health checkup by comparing the health checkup results of the user with daily measurement results or examination results. Further, in the health checkup, it is possible to confirm an improvement situation or a deterioration situation of items that are determined as "transitional measures" on the basis of daily measurement results and examination results.

Further, the health monitoring system of the present invention may notify an expert such as a doctor or a pharmacist of information on the user stored in the storage unit 130 on the basis of a request of the user, for example. The information on the user is the measurement results or examination results, the health checkup results, the questionnaire results of the user. The expert such as a doctor or a pharmacist can confirm the information on the user stored in the storage unit 130 in a lump and determine the presence or absence of the disease of the user. The health monitoring system, for example, may notify the user terminal 300 of determination results of the expert such as a doctor or a pharmacist, an advice from the expert to the user, and the like. Therefore, the user can receive the determination results, an improvement proposal, or the like of the expert.

Since the storage unit 130 stores the questionnaire results or the health checkup results and the measurement results or the examination result of the measurement device 200 in association with each other for each user as described above, it is possible to perform a detailed study on the state of health of the user on the basis of such information and to make optimal improvement proposal for a lifestyle of the user.

A method of storing and managing data is not limited to the DB and the data may be stored in various configuration files (hereinafter referred to as "configuration files") such as a definition file, a parameter file, or a temporary file. The storage unit 250 is typically realized by various recording media such as a hard disc drive (HDD), a solid state drive (SSD), and a flash memory (SD (Secure Digital) memory card). Various DBs are shown in <Data> to be described below. The above is the configuration of the server 100.

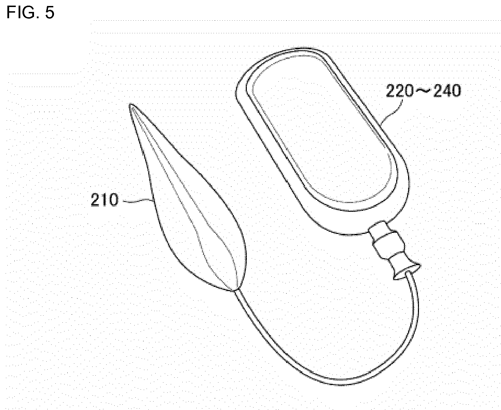

Next, a configuration of the measurement device 200 will be described. As illustrated in FIG. 3, the measurement device 200 includes the measurement unit 210, the user identification unit 220, the control unit 230, the communication unit 240, and the storage unit 250. Further, in the measurement device 200, each unit can be disposed in a plurality of devices. For example, as illustrated in FIG. 5, the measurement unit 210 can be disposed in the device as illustrated on the right of FIG. 5, whereas the user identification unit 220, the control unit 230, the communication unit 240, and the storage unit 250 can be collectively disposed in another device as illustrated on the left of FIG. 5. Accordingly, a device having only the measurement unit 210 disposed therein may be be installed, for example, in the bowl of the toilet, and the other device may be appropriately installed in a range in which there is no problem in communication, and the device is configured with general versatility with respect to a shape of the toilet.

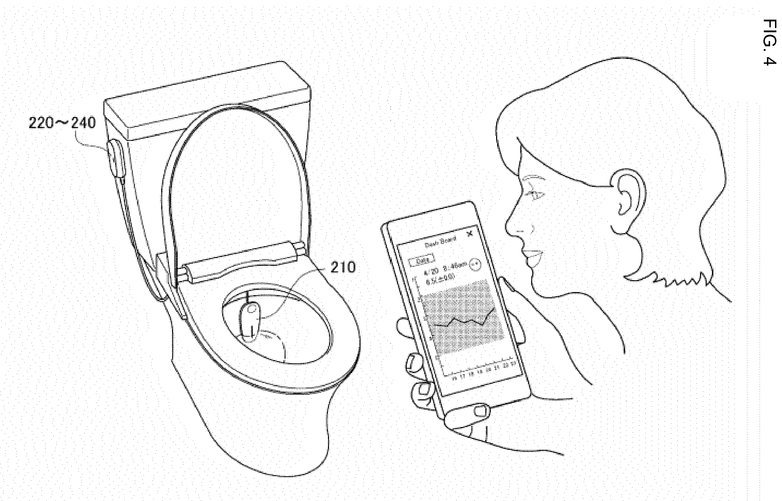

The measurement unit 210 includes an electrode unit 211, an imaging unit 212, an illuminance sensor unit 213, and a temperature measurement unit 214. For example, as illustrated in FIG. 4, the measurement unit 210 may be installed such that at least some of the electrode unit 211, the imaging unit 212, and the temperature measurement unit 214 are immersed in the reserved water inside the bowl of the toilet. When the user transfers a measurement start input by an input means included in the control unit 230 to the measurement unit 210, the measurement unit 210 can cause the electrode unit 211, the imaging unit 212, the illuminance sensor unit 213, and the temperature measurement unit 214 to start respective measurements using the transfer as a trigger.

When at least one of the temperature information (for example, the water temperature of the reserved water or the urine-containing water) generated by the electrode unit 211 or the voltage information (for example, the potential difference) generated by the temperature measurement unit 214 has reached a predetermined threshold value, the measurement unit 210 can automatically start or end each measurement of each unit constituting the measurement unit 210. Further, the measurement unit 210 can automatically start or end the measurement on the basis of a detection result of an infrared sensor (not illustrated) capable of detecting the presence or absence of a user, which is provided in the measurement device 200. Further, the measurement unit 210 may also automatically end or automatically start the measurement on the basis of a pressure sensor (not illustrated) provided in the toilet seat. Accordingly, the user can start the measurement in a normal urination action without performing an action for selecting start or end each time the measurement is started or ended, and it is possible to provide a measurement device convenient for users. It should be noted that, in a case in which the measurement is started on the basis of the temperature information, for example, the start of the measurement is determined on the basis of a change in temperature of water in the toilet before and after a urination action of the user is started. For example, when the temperature of the water in the toilet rises due to the urination of the user, the measurement is started.

Further, the measurement unit 210 may automatically start the measurement using the user identification unit 220 completing a process of identifying a user as a trigger. Further, the measurement unit 210 may set a threshold value for each measurement item, and end the measurement using acquisition of data reaching the threshold value as a trigger. Further, the measurement unit 210 may manually start or end the measurement according to an operation input from the display unit 330 of the user terminal 300. Further, a human sensor (not illustrated) may be provided in the measurement device 200, measurement may be started using detection of a sign of the person using infrared rays or the like of the human sensor as a trigger, or the measurement may be ended using detection that there is no sign of the person as a trigger. Further, the measurement unit 210 may also automatically end or automatically start the measurement on the basis of a pressure sensor (not illustrated) provided in the toilet seat. For example, the measurement unit 210 may automatically start the measurement when the pressure sensor has detected the pressure, and automatically end the measurement when the pressure sensor has not detected the pressure.

The electrode unit 211 has a function of measuring an electromotive force (a potential difference or a voltage value) due to an electrolyte and a current value flowing between the electrodes immersed in the urine-containing water with respect to a specific component in the urine which is the electrolyte using two or more electrodes to generate voltage information. Specifically, for example, the electrode unit 211 is configured of two or more electrodes, a potentiometer, and an ammeter in order to measure a concentration of the specific component in the urine. In the electrode unit 211, for example, one of the electrodes is used as a reference electrode, the other electrode is used as a working electrode, the electrodes are immersed in the urine-containing water, and an electromotive force difference between the working electrode and the reference electrode responding to the concentration (activity) of the urine component for the purpose of analysis of the urine-containing water is measured with a potentiometer. Voltage information is generated on the basis of the measurement result, and the generated voltage information is transferred to the transmission unit 242 via the control unit 230 in order for the generated voltage information to be transmitted to the server 100.

Here, the "voltage information" refers to information on the electromotive force (a potential difference or a voltage value) due to a specific component (electrolyte) in the urine that is generated using the electrodes of the electrode unit 211. It should be noted that although the example in which the ion-selective electrode method has been used is shown, an enzyme electrode method (GOD (Glucose OxiDase)) may be used or an electrode serving as a counter electrode may be added and an electrode method based on a triode may be used. Accordingly, it is possible to measure the concentration or the like of the specific component in the urine on the basis of the generated voltage information.

The imaging unit 212 has a function of causing a specific component in the urine to have a coloring reaction with a reagent or the like using a bioassay to image a reaction state. Specifically, for example, the imaging unit 212 includes the film 90 of which the color is changed according to components of urine-containing water, and an imaging means that images the film 90. Here, the "film 90" may be any material as long as a specific component in the urine can be caused to have a coloring reaction with an added reagent and can have a tape shape (for example, a shape in which a thickness of a strip is thin and that can be taken up by a reel or the like). The film 90 may be formed of a polymer component such as a synthetic resin, or may be formed of a fiber such as paper or cloth. It should be noted that it is preferable for the film 90 to be transparent. For example, when the imaging unit 212 has used the immunochromatography method as an assay method, the film 90 includes a sample pad, a conjugate pad, a test line (a detection line), a control line, a membrane, an absorption pad, and the like, but the present invention is not limited thereto. It should be noted that a configuration of the film 90 will be described below.

The sample pad is immersed in the urine-containing water so that the urine-containing water is absorbed, an RGB (Red Green Blue) value of the color developed by a coloring reaction of the test line and the control line is read by the imaging means such as a camera performing imaging, and imaging information (the read RGB value) is transferred to the transmission unit 242 via the control unit 230 in order for the imaging information to be transmitted to the server 100.

It should be noted that the server 100 measures the color developed due to a coloring reaction on the basis of the imaging information. Accordingly, it is possible to suppress a cost and measure the color, rather than reading a wavelength or the like using a spectroscope or the like. In this case, it is assumed that noise is included, but the noise can be eliminated by the correction unit 122 of the server 100.

Here, there is a problem that the related art of Japanese Unexamined Patent Application Publication No. 2013-36817 and Japanese Unexamined Patent Application Publication No. 2013-90748 cannot be applied to an examination method of adding a sample to a pad to cause an antigen-antibody reaction and form a complex, further binding the complex to a different type of antibody as a complex, and determining whether a pregnancy or a disease is positive or negative according to the reaction (for example, color development), which uses an antigen-antibody reaction such as an immunochromatographic assay method.

The health monitoring system according to the present invention further includes the film 90 of which the color is changed according to the components of the reserved water into which the urine has flowed, and the imaging unit 212 including an imaging means that images the film 90 to generate imaging information, and the correction unit corrects the imaging information on the basis of the urine information including the water volume information and the urine volume of urination, and the analysis unit 123 analyzes the urine components on the basis of the corrected imaging information. Thus, it is possible to apply the health monitoring system to an examination method using an antigen-antibody reaction such as an immunochromatographic assay method, and to perform more measurements as compared with a urination information measurement device or the like installed in a toilet in the related art.

The illuminance sensor unit 213 has a function of measuring illuminance (brightness) of a surface of the film 90 imaged by the imaging unit 212. The illuminance sensor unit 213 is configured of a light reception element such as a photodiode. For example, the illuminance sensor unit 213 converts light incident on the light reception element into a current, detects the illuminance, and transmits illuminance information to the transmission unit 242 via the control unit 230, thereby transferring the illuminance information to the server 100. It should be noted that the server 100 can correct the measurement results of the color using the illuminance information. Accordingly, it is possible to obtain the color measurement results in consideration of the illuminance due to the illumination, and to accurately analyze a reaction state of a specific component for the purpose of urine analysis.

The temperature measurement unit 214 has a function of measuring a temperature of the reserved water inside the bowl of the toilet or a temperature of the urine-containing water to generate water temperature information. The temperature measurement unit 214 is configured of, for example, a thermistor, an oscillator, and a counter. The temperature measurement unit 214 outputs a change in a resistance value due to a temperature change using the thermistor, converts the change in the resistance value into a frequency using the oscillator, and measures the frequency using the counter, thereby measuring the temperature. The temperature measurement unit 214 transfers the water temperature information to the transmission unit 242 via the control unit 230 in order to transmit the water temperature information to the server 100.

The user identification unit 220 has a function of identifying a user who is a target that the health monitoring system 500 monitors using toilet. For example, as illustrated in FIG. 4, the user identification unit 220 is connected to the measurement unit 210 by a wire such as a cable, and may include an adsorption means with respect to a pottery device such as a tank, for mounting in a tank that stores washing water, and may include another attachment means.

Specifically, for example, the user identification unit 220 reads information (for example, QR code (registered trademark)) for uniquely identifying a user, which is output by a health monitoring application installed in the terminal 300 possessed by the user (hereinafter, the information for identifying the user is referred to as "user identification information"), magnetic information for uniquely identifying a user of an integrated circuit (IC) card possessed by the user, and information for uniquely identifying a user of Worldwide Interoperability for Microwave Access (WiMAX) or wireless LAN such as Wireless Fidelity (WiFi) or Bluetooth (for example, received signal strength information or radio wave reception strength information), to identify the user.

The user identification unit 220, for example, reads a QR code or a barcode displayed on the display unit 330 of the user terminal 300 to identify the user. In this case, the user identification unit 220 has a function of reading (scanning) the QR code and the barcode. The user, for example, displays the QR code or the barcode on the display unit 330 of the user terminal 300 and causes the user identification unit 220 of the measurement device 200 to read the QR code or the barcode.

Further, the user identification unit 220 may read magnetic information from an RF tag included in an IC card or a user terminal possessed by the user and identify the user using a radio frequency identifier (RFID) technology. The RFID technology is a technology for exchanging information from an RF tag with ID information embedded therein using short-distance wireless communication using electromagnetic field, radio waves, or the like. The RF tag may be configured of a circuit board on which a plurality of electronic elements are mounted or may be realized by an integrated circuit (IC). For example, the user identification unit 220 reads magnetic information included in the IC card or the user terminal possessed by the user using short-distance wireless communication and identifies the user. The reading function may be of a contact type or a non-contact type. The user, for example, brings the IC card including the magnetic information or the user terminal 300 to be closer to or approach the user identification unit 220 of the measurement device 200, thereby causing the user identification unit 220 to read the magnetic information.

Further, the user identification unit 220 may receive user identification information from the user terminal 300 using wireless communication such as WiMAX, WiFi, or Bluetooth. The user identification unit 220 is not limited to such wireless communication and may receive user identification information from the user terminal 300 through wireless communication such as LTE or CDMA, for example. In this case, the user may operate the user terminal 300 to transmit the user identification information to the measurement device 200. Further, when the user terminal 300 possessed by the user is located in a predetermined area (for example, inside a toilet) or the like, the user terminal 300 may be configured to transmit the user identification information to the measurement device 200 automatically (without an operation of the user).

Accordingly, it is possible to automatically perform identification of the user by moving the user terminal 300 or the IC card to the user identification unit 220, to automatically identify the network and identify a specific institution (for example, a company, a hospital, or a school), and to simply identify the user or the specific institution without inputting, through an operation, information for identifying the user or information for identifying the specific institution each time each time the user uses the toilet.

Further, the user identification unit 220 may include a measurement unit 221. The measurement unit 221, for example, measures a weight (Kg weight) of the user against the toilet seat in the case of a western style toilet, and stores information on the measured weight (hereinafter referred to as "weight information") of each user to the storage unit 250. The user identification unit 220 identifies the user on the basis of the weight information and generates user identification information. In addition, the user identification unit 220 may perform identification of a user using a face recognition sensor that performs face authentication, a posture detection sensor that performs posture detection, a pulse measurement means that performs pulse measurement of a user, a blood pressure measurement means that performs blood pressure measurement of a user, a body fat percentage measurement means that performs body fat percentage measurement of a user, and a muscle mass measurement means that performs measurement of a muscle mass of a user.

These pieces of the user identification information may be transmitted to the server 100 together with a set of the water temperature information, the voltage information, the user identification information, the illuminance information, and the imaging information or may be transmitted at a timing of the identification. The user identification unit 220 transmits the information to the transmission unit 242 via the control unit 230 in order to transmit the information to the server 100. Accordingly, it is possible to automatically perform the identification of the user as part of a usual urination action, and to simply identify the user without inputting the information for identifying the user each time the user uses the toilet.

The control unit 230 is a processor having a function of controlling each unit of the measurement device 200. Further, the control unit 230 may include the input means (not illustrated) that allows the user to manually select the start of each measurement relating to urination. The control unit 230 transfers a measurement start input by the input unit to the measurement unit 210.

The communication unit 240 includes a reception unit 241 and a transmission unit 242 and has a function of executing communication with the server 100 and each user terminal 300 via the network 400. The communication may be any of wired communication and wireless communication (for example, communication schemes such as Wi-Fi (Wireless Fidelity), BLE (Bluetooth Low Energy), ZigBee, or the like). Any communication protocol may be used as long as mutual communication can be executed.

The reception unit 241 has a function of receiving control data or the like from each server 100 and each user terminal 300 via the network 400 and transferring the control data or the like to the control unit 120 under the control of the control unit 230. Specifically, the reception unit 241 receives user information (for example, ID information) stored in the storage unit 130 for control of the user identification unit 220 and, for example, dynamic parameter data necessary for measurement and imaging of the measurement unit 210 and identification of the user identification unit 220 from the server 100, and transfers the user information and the received dynamic parameter data to the control unit 230.

The transmission unit 242 has a function of transmitting measurement data or the like to the server 100 and each user terminal 300 via the network 400 under the control of the control unit 230 Specifically, for example, the transmission unit 242 transmits the water temperature information, the voltage information, the user identification information (including the measurement information), the illuminance information, and the imaging information to the server 100 or each user terminal 300. It should be noted that examples of the transmission timing of the transmission unit 242 may be, for example, (1) immediate after measurement (for example, using transfer of measurement data from the measurement unit 210 as a trigger), (2) periodically (for example, at certain intervals determined in consideration of a life rhythm of the user or a capacity of the storage unit 250), and (3) when a threshold value set in the storage capacity of the storage unit 250 is reached.

The storage unit 250 has a function of storing various programs, data, and parameters necessary for an operation of the measurement device 200. Specifically, for example, the storage unit 250 stores user information, and parameters necessary for operations of the measurement unit 210, the user identification unit 220, the control unit 230, and the communication unit 240. The storage unit 250 is typically realized by various recording media such as a hard disc drive (HDD), a solid state drive (SSD), and a flash memory (SD (Secure Digital) memory card). The above is the configuration of the measurement device 200.

Next, a configuration of the user terminal 300 will be described. As illustrated in FIG. 3, the user terminal 300 includes a communication unit 310, a control unit 320, a display unit 330, and a storage unit 340. Each unit of the user terminal 300 may be included in a health monitoring application or may be incorporated in a circuit of the user terminal 300.

The communication unit 310 includes a reception unit 311 and a transmission unit 312 and has a function of executing communication with the server 100 and the measurement device 200 via the network 400. The communication may be either wired or wireless, and any communication protocol may be used as long as mutual communication can be executed.

The reception unit 311 has a function of receiving display data or the like from each server 100 and each measurement device 200 via the network 400 under control of the control unit 320 and transferring the display data or the like to the control unit 320. Specifically, for example, the reception unit 311 receives display information including urine examination results from the server 100, receives user information (for example, ID information) stored in the storage unit 130 for control of the user identification unit 220 and, for example, dynamic parameter data necessary for measurement and imaging of the measurement unit 210 and identification of the user identification unit 220 from the server 100, and transfers the display information and the dynamic parameter data to the control unit 230.

The transmission unit 312 has a function of transmitting, for example, user identification information such as input information and QR code information input by the user from the display unit 330 to the server 100 and each measurement device 200 via the network 400 under the control of the control unit 320.

The control unit 320 is a processor having a function of controlling each unit of the user terminal 300. Further, when the input result is transferred from the display unit 330 and when the inference results are transferred from the inference unit 124, the control unit 320 generates display data for displaying the results on the display unit 330 of the user terminal 300 as a text, a table, or a graph. In order to transmit the generated display data to the user terminal 300, the control unit 120 transfers the generated display data to the transmission unit 112.

The display unit 330 has a function of displaying the display data or the like received from the server 100 or the measurement device 200. Specifically, for example, as illustrated in FIG. 4, the display unit 330 displays display data indicating monitoring results such as a measurement value related to measured urination, a result of measurement of normality or abnormality, an analysis result related to analyzed urine components, and a result of inference as to whether an inferred disease is positive or negative using text, table, graph, or the like. The results may be displayed in units of display units designated by a user, such as a day, a week, or a month. Further, the display unit 330 may include an input means for the user and cause the user to input, for example, user identification information (for example, name, age, sex, height, weight, or the like).

The storage unit 340 has a function of storing various programs, data, and parameters necessary for an operation of the user terminal 300. Specifically, for example, the storage unit 340 stores user identification information, and parameters necessary for operations of the communication unit 310, the control unit 320, the display unit 330, and the storage unit 340. The storage unit 250 is typically realized by various recording media such as a hard disc drive (HDD), a solid state drive (SSD), and a flash memory (SD (Secure Digital) memory card). The above is the configuration of the user terminal 300.

FIG. 4 is a diagram schematically illustrating an example of an overview of the health monitoring system 500. This example is an example in which the measurement device 200 is installed in a western style toilet and the health monitoring system is used. It should be noted that a type of a toilet is not limited to the western style toilet, and any type of toilet may be used as long as the toilet is a toilet in which there is reserved water for washing and drainage when the toilet is a Japanese style toilet or the like. As illustrated in FIG. 4, a portion (for example, the measurement unit 210) that measures information on the reserved water and the urine-containing water of the measurement device 200 is installed in a device to be immersed in the reserved water in the bowl of the toilet, other portion not required to be immersed in the reserved water (for example, the user identification unit 220, the control unit 230, and the communication unit 240) are disposed in another device. This device, for example, may be installed in a tank. Further, a device in which the user identification unit 220 may be a device that can be disposed at a position at which the user terminal 300 or the like is held so that the device can read the QR code information output from the user terminal 300 possessed by the user and the information output by the IC card. Accordingly, the measurement can be performed after the user is identified without input of the user identification information to the measurement device 200 each time the user uses the device.

Next, hereafter, an internal structure of the measurement unit 210 constituting the measurement device 200 will be described.

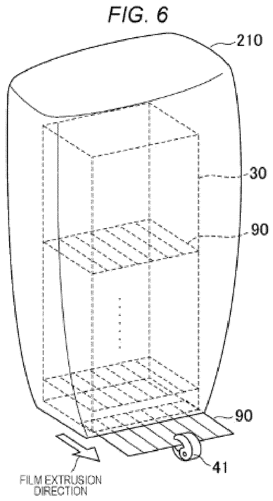

FIG. 6 is a diagram schematically illustrating an example of an internal structure of the measurement unit 210 according to Embodiment 1. Specifically, FIG. 6 illustrates a configuration example of the film 90 in a case in which the film 90 is stored in the measurement unit 210. As illustrated in FIG. 6, the measurement unit 210 includes a cartridge 30 in which a plurality of films 90 are stacked and stored. The cartridge 30 can be attached to and detached from the measurement unit 210. When the film 90 runs out in the cartridge 30, the cartridge 30 can be replaced with a new cartridge 30 in which the films 90 are stored.

For example, a plurality of about 30 films 90 are stacked and stored in the cartridge 30. It should be noted that any number of films 90 can be stored in the cartridge 30.

As illustrated in FIG. 6, the film 90 located at a bottom of the cartridge 30 is extruded to the outside of the measurement unit 210 at the start of measurement. Accordingly, the film 90 can be used for measurement. Thus, Embodiment 1 of the present invention is an example of a strip type in which the film 90 is taken out one by one. The films 90 are sequentially immersed in the reserved water or the urine-containing water, and a reagent placed on the film 90 is caused to have a coloring reaction.

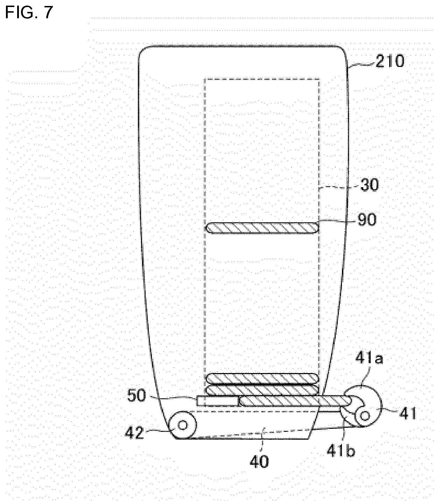

FIG. 7 is a diagram schematically illustrating another example of an internal structure of the measurement unit 210 according to Embodiment 1.

As illustrated in FIG. 7, the measurement unit 210 includes an extrusion mechanism 50 for extruding the film 90 at the bottom of the cartridge 30. The extrusion mechanism 50 extrudes the film 90 located at the bottom of the cartridge 30 to the outside of the measurement unit 210 each time the measurement is started. The extrusion mechanism 50 may have any configuration as long as the stacked and stored film 90 can be extruded. The extrusion mechanism 50 applies a force in an extrusion direction to one side of the film 90 to extrude the film 90 in the extrusion direction.

The extrusion mechanism 50 may extrude all the films 90 to the outside of the measurement unit 210 or may extrude some of the films 90. For example, the extrusion mechanism 31 extrudes the film 90 located at the bottom of the cartridge 30 to the outside of the measurement unit 210 to a position at which the film 90 can be clamped by the clamping of the arm 40. In response to a request to start measurement, for example, the measurement unit 210 operates the extrusion mechanism 50 to extracts the film 90 to the outside of the measurement unit 210.

Further, as illustrated in FIG. 7, the measurement unit 210 includes an arm 40. The arm 40 includes a clamping portion 41 for clamping the film 90 at one end thereof. The clamping portion 41 clamps the film 90 extruded to the outside of the measurement unit 210. As illustrated in FIG. 7, the clamping portion 41 has, for example, a shape like a beak, and clamps the film 90 using tips of one clamping member 41a and the other clamping member 41b. It should be noted that the clamping portion 41 may have any shape or configuration as long as the film 90 can be clamped, and may have, for example, a shape like tweezers.

Further, a tip of the arm 40 is not required to be the clamping portion 41. For example, the tip may have a shape like a needle. In this case, the film 90 may be punctured (penetrated) by the tip of the arm 40 so that the film 90 is held at one end of the arm 40.

Further, the other end of the arm 40 is connected to a rotation mechanism 42. The rotation mechanism 42 can move the clamping portion 41 that is one end of the arm 40 in a vertical direction by rotating the arm 40 around the rotation mechanism 42.

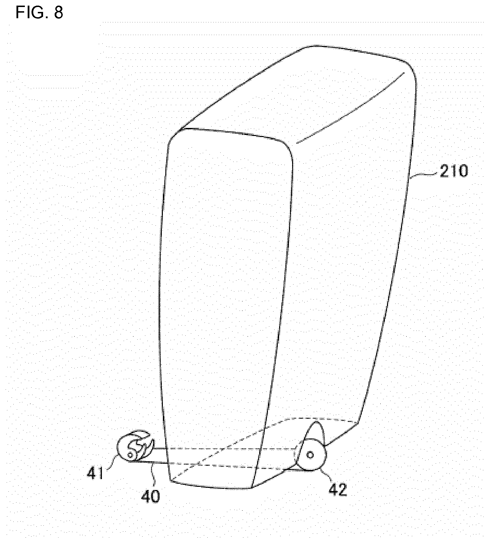

FIG. 8 is a diagram schematically illustrating a configuration example of the measurement unit 210 constituting the measurement device 200 according to Embodiment 1. As illustrated in FIG. 8, the measurement unit 210 includes, for example, a rotation mechanism 42 near a lower center of a back surface of the measurement unit 210. As described above, one end of the arm 40 is connected to the rotation mechanism 42, and the clamping portion 41 that is the other end of the arm 40 can be moved in the vertical direction.

The measurement unit 210 can move the film 90 in a vertical direction by operating the rotation mechanism 42 after the clamping portion 41 of the arm 40 clamps the film 90.

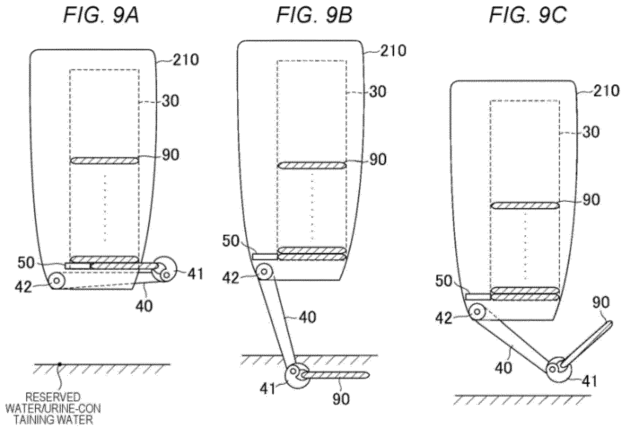

FIG. 9 is a diagram schematically illustrating a state in which the clamping portion 41 of the arm 40 according to Embodiment 1 moves in the vertical direction.

FIG. 9(a) illustrates a position of the clamping portion 41 in a case in which the clamping portion 41 of the arm 40 clamps the film 90. As illustrated in FIG. 9(a), the clamping portion 41 clamps the film 90, for example, above the reserved water or the urine-containing water.

Subsequently, as illustrated in FIG. 9(b), the clamping portion 41 of the arm 40 moves in a downward direction by operating the rotation mechanism 42 of the arm 40 after the clamping portion 41 clamps the film 90. Accordingly, in the measurement unit 210, the film 90 clamped by the clamping portion 41 is moved in a downward direction together with the clamping portion 41 so that the film 90 is immersed in the reserved water or the urine-containing water. The arm 40 of the measurement unit 210 stops in a state in which the film 90 is immersed in the reserved water or the urine-containing water, and causes the reagent placed on the film 90 to have a coloring reaction.

When the coloring reaction of the reagent placed on the film 90 is completed, the measurement unit 210 causes the rotation mechanism 42 of the arm 40 to operate again and moves the clamping portion 41 of the arm 40 in an upward direction. The measurement unit 210 moves the clamping portion 41 of the arm 40 in the upward direction up to a position at which an imaging means (not illustrated) of the imaging unit 212 can image the film 90 after the reaction. As illustrated in FIG. 9(c), the measurement unit 210 moves the clamping portion 41, for example, up to a position above the reserved water or the urine-containing water.

The measurement unit 210 images the coloring reaction of the reagent placed on the film 90 using an imaging means (not illustrated) of the imaging unit 212 included in the measurement unit 210.

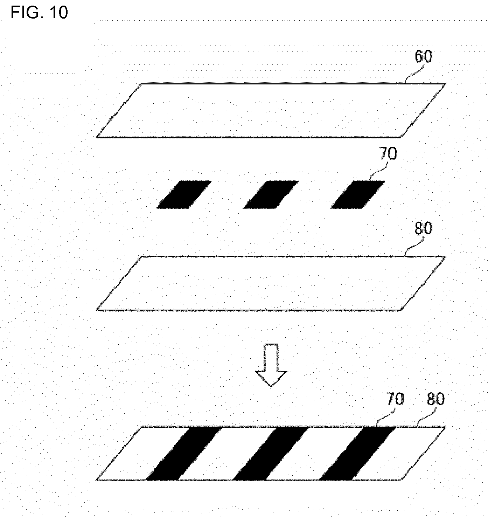

Next, a configuration of the film 90 constituting the imaging unit 212 and a reagent 70 placed on the film 90 will be described. FIG. 10 is a diagram schematically illustrating an example of the configuration of the film 90 and the reagent 70 constituting the imaging unit 212. As illustrated in FIG. 10, for the film 90, the film 90 constituting the imaging unit 212 can be configured by sandwiching the reagent between the top film 60 for protecting a surface of the reagent 70 and a support film 80 for placing the reagent (for supporting the reagent) using the top film 60 and the support film 80. It is conceivable to peel off the top film 60 immediately before the measurement by (1) dissolving the top film 60 at the time of the measurement using a water-soluble film and (2) incorporating a mechanism for peeling off the top film 60 into the measurement unit 210. Using (1) or (2), it is possible to protect the reagent until immediately before measurement and to prevent deterioration of the reagent. Further, it is also possible to prevent the deterioration of the reagent by minimizing the amount of air with which the film 90 comes in contact until immediately before the measurement by (3) configurating the cartridge 30 in which the film 90 has been stored to have a highly air-tight structure without using the top film 60.

After the imaging of the coloring reaction of the reagent in the imaging unit 212 is completed, the measurement unit 210 releases the film 90 from the clamping portion 41 (stops clamping of the film 90). Therefore, the film 90 falls into the reserved water or the urine-containing water. The film 90 is water-soluble, and is dissolved in the reserved water or the urine-containing water by falling into the reserved water or the urine-containing water. Further, the film 90 is discarded together with the reserved water or the urine-containing water at the time of flushing (for example, at the time of washing the toilet by flushing urination with water). It should be noted that when the tip of the arm 40 has a needle-like shape, the film 90 may be moved in a downward direction again up to a position at which the film 90 is immersed in the reserved water or the urine-containing water after the imaging of the coloring reaction is completed, and the water-soluble film 90 may be dissolved.

### <Data>

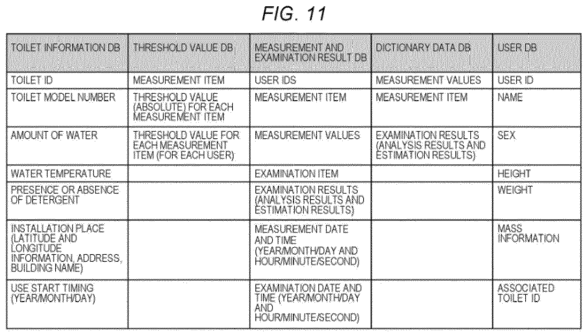

In this embodiment, an example of a data configuration of various DBs stored in the storage unit 130 will be described herein by way of example with reference to FIG. 11. It should be noted that a storage destination of each of the various DBs is not limited to the storage unit 130 of the server 100, and may be the storage unit 250 of the measurement device 200 or may be the storage unit 340 of the user terminal 300. Further, it should be noted that the data configuration may be appropriately changed according to the functional configuration, processing content, and the like of the server 100.

First, a toilet information DB is a DB that stores information on a toilet. The toilet information DB includes, for example, a toilet model number, the amount of water (water level, mass, volume, and the like of the reserved water), a water temperature (water temperature information of the reserved water), presence and absence of a detergent, an installation place (latitude and longitude information, address, building name, or the like), and a use start timing (toilet use start timing). Further, the toilet information DB may further include information on toilet environment (not illustrated) such as information on the amount of detergent or the like or component information of the detergent or the like. The toilet information DB holds records in units of toilets. It should be noted that information associated with the toilet model number (for example, shape information of the bowl of the toilet, or water volume information of the toilet) may be held in the DB or may be searched for and acquired using a network system such as the Internet each time instead of being stored in the DB.

Next, the threshold value DB is a DB that stores a threshold value that serves as a reference for determining whether the measurement result indicates positive or negative, or normal or abnormal. The threshold value DB includes, for example, information such as a measurement item, a threshold value (absolute) for each measurement item (a reference value as an absolute index for each measurement item), and a threshold value for each measurement item (for each user) (a reference value as a personalized index for each user for each measurement item).

Next, the measurement and examination result DB is a DB that stores measurement results and examination results for each user. The measurement and examination result DB includes, for example, information such as a user IDs (user identification information), measurement items, measurement values, examination items, examination results (analysis results and inference results), and measurement date and time (year/month/day and hour/minute/second), examination date and time (year/month/day and hour/minute/second).

Next, the dictionary data DB is a DB that stores dictionary data. The dictionary data DB includes, for example, information such as measurement values, examination results (analysis results and inference results). The dictionary data DB identifies a feature vector created from measurement values as so-called teacher data in machine learning. It should be noted that the dictionary data to be stored in the dictionary data DB may be defined and stored in the configuration file. Using the configuration file, a speed of a process of reading and updating the dictionary data can be considered to be improved as compared with a case in which a DB is used.

Next, the user DB is a DB that stores information for uniquely identifying the user. The user DB includes, for example, information such as a user ID (information of uniquely given alphanumeric characters), a name, sex, height, and weight of a user information, mass information measured by the measurement device 200, and toilet IDs of one or more toilets associated with the user. The above is the data configuration of various DBs.

Next, a data configuration example of association between measurement and analysis results of the health monitoring system 500 and information such as disease will be described with reference to FIG. 12. FIG. 12 is a data conceptual diagram illustrating the association. For example, an albumin component in the urination is used as input information, a degree of coloring of the film 90 due to a coloring reaction of the film 90 to a reagent or the like is measured according to the input information in the imaging unit 212 using an immunochromatographic method, an albumin concentration in the urine is analyzed from the degree of coloring, and it is determined, for example, whether or not an analysis result exceeds a corresponding threshold value. The user infers whether diabetes is positive or negative on the basis of a result of the determination.

### <Operation>

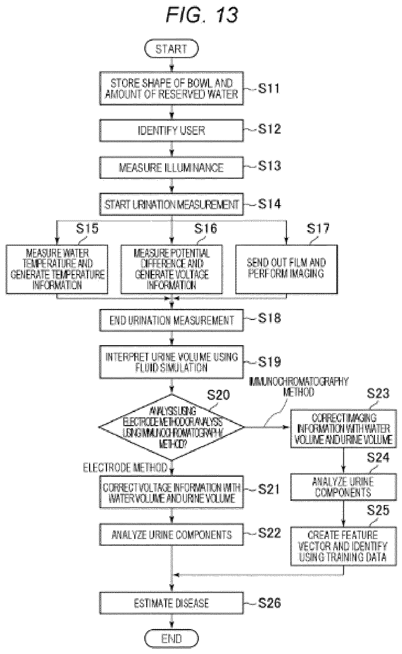

FIG. 13 is a flowchart showing an example of a process that is executed by the health monitoring system 500.

The storage unit 130 stores shape information of the bowl of the toilet, water volume information of the reserved water, and water temperature information of the reserved water as initial settings in advance or each time measurement is performed (step S11). The user identification unit 220 identifies the user using the IC card, the user terminal 300, or the like (step S12). After this step, the measurement unit 210 may measure the water temperature of the reserved water (not illustrated). The illuminance sensor unit 213 measures the illuminance of the surface of the film 90 (step S13). When the user manually transfers a measurement start input by the input means included in the control unit 230 to the measurement unit 210, the measurement unit 210 starts each measurement (step S14). It should be noted that this step can be omitted when the electrode unit 211, the imaging unit 212, and the temperature measurement unit 214 automatically start the measurement.

In a case in which the measurement is started automatically or manually, for example, when a temperature to be measured reaches a certain threshold value, the temperature measurement unit 214 measures the temperature of the reserved water or the urine-containing water and generates water temperature information (step S15). In a case in which the measurement is started automatically or manually, for example, when a potential difference to be measured reaches a certain threshold value, the electrode unit 211 measures a potential difference between the electrodes and generates voltage information (step S16). In a case in which the measurement is started automatically or manually, the measurement unit 210 operates the extrusion mechanism 50 to extrude the film 90 located at the bottom of the cartridge 30 and to perform imaging (step S17). It should be noted that in step S17, the measurement unit 210 first operates the clamping portion 41 of the arm 40, and clamps the extruded film 90 using the clamping portion 41. When the clamping portion 41 clamps the film 90, the measurement unit 210 operates the rotation mechanism 42 to move the clamping portion 41 in a downward direction. Accordingly, the film 90 clamped by the clamping portion 41 is immersed in the reserved water or the urine-containing water. The measurement unit 210 stops in a state in which the film 90 is immersed in the reserved water or the urine-containing water, and causes the reagent placed on the film 90 to have the coloring reaction. Thereafter, the measurement unit 210 operates the rotation mechanism 42 to move the clamping portion 41 of the arm 40 in an upward direction to a position at which an imaging means (not illustrated) of the imaging unit 212 can image the film 90 after the reaction. The measurement unit 210 sends out the film 90 so that a sample pad portion of the film 90 is immersed in the reserved water or the urine-containing water, and images an RGB luminance signal of the test line and the control line using the imaging means such as a camera (step S21). It should be noted that after the imaging of the imaging means of the imaging unit 212, the clamping portion 41 of the arm 40 may release the film 90 and drop the film 90 into the reserved water or the urine-containing water.

The temperature measurement unit 214 automatically ends the measurement, for example, when the measured temperature reaches the certain threshold value, and the electrode unit 211 automatically ends the measurement when the measured potential difference reaches the certain threshold value (step S18).

The interpretation unit 121 interprets the fluid using the fluid model obtained by modeling the fluid flowing around the measurement unit 210 on the basis of, for example, the shape information, the water volume information, and the water temperature information to interpret (calculate) the urine volume (step S19). When the measurement value is analyzed using the electrode method (the electrode method in step S20), the correction unit 122 calculates the degree of dilution on the basis of the analyzed urine volume information and the water volume information, and corrects the voltage information on the basis of the degree of dilution (step S21). The analysis unit 123 analyzes the urine components on the basis of the voltage information (after correction) (step S22).

When the measurement value is analyzed using an immunochromatography method (the immunochromatography method in step S20), the correction unit 122 calculates the degree of dilution on the basis of the analyzed urine volume information and the water volume information, and corrects the imaging information on the basis of the degree of dilution (step S23). It should be noted that, in this step, the correction unit 122 may correct the imaging information on the basis of the illuminance information in addition to the degree of dilution. The analysis unit 123 analyzes the urine components on the basis of the imaging information (after correction) (step S24). The analysis unit 123 creates a feature vector on the basis of an analysis result and identifies the created feature vector using training data (the dictionary data) (step S25). The inference unit 124 infers a disease of the user on the basis of the interpreted urination information of the urination (for example, the analyzed urine components) (step S26).

### (Embodiment 2)

Embodiment 2 is an embodiment in which a shape or material of the measurement unit 210 is devised so that it is difficult for an object such as a toilet paper or the like to be attached to the measurement unit 210. It should be noted that a configuration described in Embodiment 2 can be applied to any of Embodiment 1 and the following embodiments.

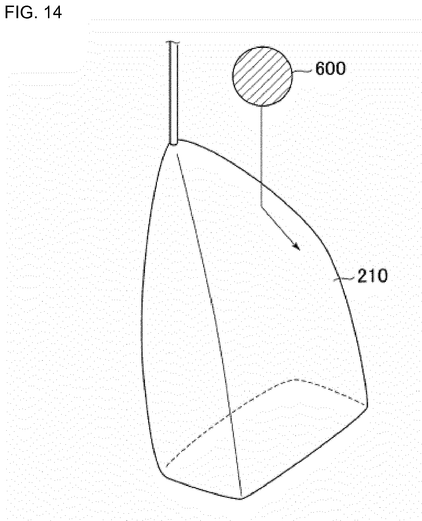

FIG. 14 is a diagram schematically illustrating an example of an overview of the measurement unit 210 in Embodiment 2. As illustrated in FIG. 14, the measurement unit 210 has, for example, a substantially streamlined shape having an upper portion as a tip. Further, the measurement unit 210 may have, for example, a substantially triangular pyramid or a substantially conical shape having an upper portion as a tip. Further, the measurement unit 210 may have a trapezoidal shape having an upper portion as a tip. Thus, the measurement unit 210, for example, has a tapered shape in which a housing is narrowed from a lower portion to an upper portion.

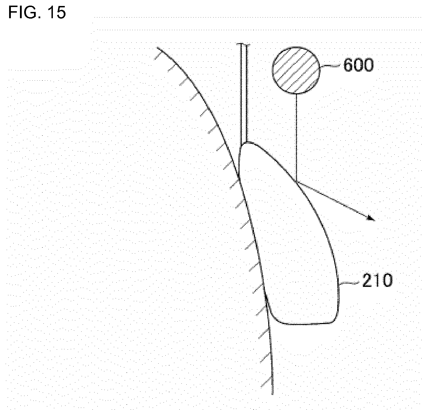

FIG. 15 is a diagram schematically illustrating another example of the overview of the measurement unit 210 in Embodiment 2. As illustrated in FIG. 15, the measurement unit 210 is, for example, a substantially streamlined shape having an upper portion in a cross section as a tip. Therefore, the measurement unit 210 can repel an object from the upper portion in front of the measurement unit 210, as illustrated in FIG. 15. Therefore, it is difficult for an object such as a toilet paper hitting from the upper portion to be attached to the measurement unit 210. Further, since a side surface portion of the measurement unit 210 has a shaped tapered from a lower portion to the upper portion, the object can be repelled even when an object hits the side surface portion, and it is difficult for the object to be attached to the measurement unit 210.

It should be noted that the measurement unit 210 may have any shape as long as the shape is a shape to which it is difficult for an object to be attached from the upper portion.

Further, the measurement unit 210 of Embodiment 2 may be coated with a material such that it is difficult for a toilet paper or the like to be attached. For example, the coating material is an affinity coating. In the measurement unit 210 subjected to affinity coating, water enters between the affinity coating and a dirt so that the dirt is washed off. That is, the measurement unit 210 subjected to affinity coating has a self-washing action. Further, the coating material may be, for example, a fluororesin. Since the fluorineresin has a small coefficient of friction, it is difficult for an object to be attached to the measurement unit 210. Further, it is difficult for not only to an object such as the toilet paper but also oil, dirt, or the like to be attached to the measurement unit 210 coated with the fluororesin. Therefore, it is possible to keep the measurement unit 210 clean.

Further, the surface of the measurement unit 210 of Embodiment 2 may be formed in a fine irregularity shape. For example, the surface of the measurement unit 210 is formed with irregularities in a sinusoidal shape. Further, the surface of the measurement unit 210 may be formed with, for example, irregularities in which an inclination of an inclined surface of a convex portion is less than 10 degrees. It should be noted that the inclination of the inclined surface of the convex portion need not be less than 10 degrees, and may be any inclination as long as it is difficult for an object to be attached. Since the surface of the measurement unit 210 is the irregularity shape, it is difficult for an object such as a toilet paper, grease, and dirt to be attached to the measurement unit 210.

### (Embodiment 3)

In Embodiment 3, a rotation mechanism is provided on the back surface of the measurement unit 210, and the measurement unit 210 can be tilted laterally with a flow of water. It should be noted that a configuration to be described in Embodiment 3 can be applied to any of Embodiment 1, Embodiment 2, and the following embodiments.

One method of washing the inside of the toilet with washing water (water, rainwater, or the like) is tornado washing. In the tornado washing, washing water flows laterally from the top of the toilet, and the washing water flows while swirling inside the toilet, thereby washing the inside of the toilet. In the tornado washing, it is possible to reduce the amount of washing water to be used for washing the inside of the toilet since the washing water flows vigorously inside the toilet.

Thus, when the inside of the toilet is washed using tornado washing, it is necessary for the washing water to flow while swirling. However, when the measurement unit 210 is set in the toilet, the flow of the washing water is likely to be obstructed by the measurement unit 210. In such a case, washing of the inside of the toilet using the washing water is suppressed.

Therefore, in Embodiment 3, since the measurement unit 210 can rotate along the flow of the washing water in the tornado washing by enabling the measurement unit 210 to rotate around a part of the back surface, the flow of the washing water is prevented from being obstructed.

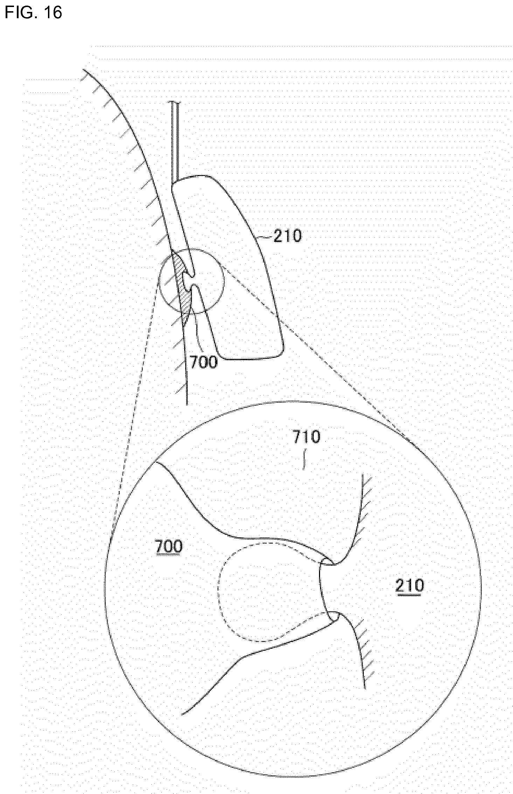

FIG. 16 is a diagram schematically illustrating a configuration example of the measurement unit 210 constituting the measurement device 200 according to Embodiment 3. As illustrated in FIG. 16, a portion of the back surface of the measurement unit 210 is rotatably connected to a fixing portion 700 fixed to a wall surface of the toilet. The measurement unit 210 includes, for example, a protrusion at a center of the back surface thereof and is rotatably connected to a sucker 700 via the protrusion.

As illustrated in FIG. 16, the measurement unit 210 includes a rotation mechanism 710. Specifically, the protrusion on the back surface of the measurement unit 210 is fitted into a recessed portion provided in the fixing portion 700, such that the measurement unit 210 can rotate around the protrusion.

It should be noted that the rotation mechanism illustrated in FIG. 16 is an example, and the rotation mechanism 710 may be in any form.

As illustrated in FIG. 16, the fixing portion 700 is, for example, a semi-cylindrical rubber sucker, and can be fixed to the wall surface of the toilet. It should be noted that the fixed portion 700 is not necessarily the rubber sucker, and may be fixed to the wall surface of the toilet in any form. For example, the fixing portion 700 may be provided as a part of the toilet in advance. Further, the fixing portion 700 may be fixed to the wall surface of the toilet with an adhesive or the like, for example.

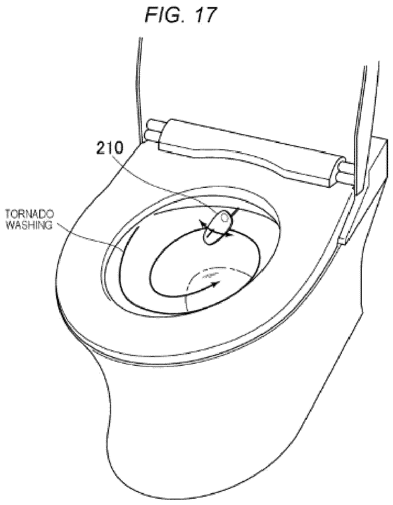

FIG. 17 is a schematic diagram illustrating a use aspect of the measurement unit 210 in Embodiment 3. As illustrated in FIG. 17, in a case in which the toilet is washed by so-called tornado washing, the measurement unit 210 can rotate in a flowing direction of washing water by the rotation mechanism illustrated in FIG. 16. As illustrated in FIG. 17, when the washing water flows from a lateral direction of the measurement unit 210, the measurement unit 210 rotates in the flowing direction (a left direction in FIG. 17) of the washing water. Therefore, it is possible to reduce suppression of washing of the inside of the toilet using the washing water without obstructing a flow of washing water.

It should be noted that since the measurement unit 210 is freely rotatable with respect to the wall surface of the toilet through the rotation mechanism 710, the measurement unit 210 rotates greatly when a momentum of a flow of washing water is strong and, on the other hand, rotates weakly when the momentum of the flow is weak. Thus, the measurement unit 210 can change a rotation angle according to a strength of the washing water.

As described above, a part of the back surface of the measurement unit 210 is fixed to the fixing unit 700 adhered to the wall surface inside the toilet, such that the measurement unit 210 can rotate along the flow of washing water in the tornado washing. Accordingly, it is possible to prevent the flow of the washing water from being obstructed.

Since the part of the back surface of the measurement unit 210 is rotatably fixed to the fixing portion 700, it is also possible to prevent the measurement unit 210 from being reversed due to the flow of the washing water. When there is no fixing portion 700, there is concern that the measurement unit 210 irregularly greatly shakes due to the flow of washing water, and the front surface and the back surface are likely to be reversed. In this case, since the measurement of the measurement unit 210 is not correctly performed, it is necessary for the user to dispose the measurement unit 210 again in a correct direction.

On the other hand, since the back surface of the measurement unit 210 is rotatably fixed to the wall surface of the toilet due to the presence of the fixing portion 700 as illustrated in FIG. 16, at least, the front surface and the back surface are not reversed in the measurement unit 210. Therefore, there is also an effect that the user does not need to fix the disposition of the measurement unit 210 in which the front surface and the back surface have been reversed.

It should be noted that, in Embodiment 3, the measurement device 200 does not necessarily need to include the fixing portion 700. The measurement unit 210 may be configured to be hung in an upper portion thereof by a cable, a string, or the like. In this case, it is preferable for the measurement device 200 to be hung by a cable, a string, or the like so that a main body of the measurement device 200 swings laterally without resisting the flow of the water for the tornado washing. Further, it is preferable for the measurement device 200 to have a weight so that the measurement device 200 can move laterally according to the flow of the washing water.

Further, in FIG. 16, the measurement device 200 is included on the front surface of the reserved water in the toilet, but the position of the measurement device 200 is not limited to the front surface but may be any position. In this regard, according to a type of toilet, a force for draining the reserved water (pulling force) increases due to swirling of the water caused by tornado washing at a place at which the reserved water in the toilet is deepest. In the tornado washing, it is possible to efficiently excrete an excrement using such a pulling force. Therefore, in such a type of toilet, it is preferable for the measurement device 200 to be attached to a place other than the place at which the reserved water in the toilet is deepest so as not to obstruct the pulling force. For example, it is preferable for the measurement device 200 to be attached to a side surface on the lateral side of a portion of the reserved water in the toilet or to a front side surface of the portion of the reserved water opposite to an attachment position of the measurement device 200 in FIG. 16. Accordingly, it is possible to prevent the measurement device 200 from obstructing the flow of the washing water in the tornado washing and to reduce the amount of washing water to be used for washing of the inside of the toilet.

As described above, in Embodiment 3 of the present invention, the measurement unit 210 is provided not to obstruct the flow of the washing water in the tornado washing, thereby preventing the flow of the washing water from being obstructed.

### (Embodiment 4)

Embodiment 4 is an embodiment in which a weight of the user sitting on the toilet seat can be measured by attaching the pressure sensor 800 to the toilet seat of the toilet. It should be noted that a configuration that will be described in Embodiment 4 can be applied to any of Embodiment 1, Embodiment 2, and Embodiment 3.

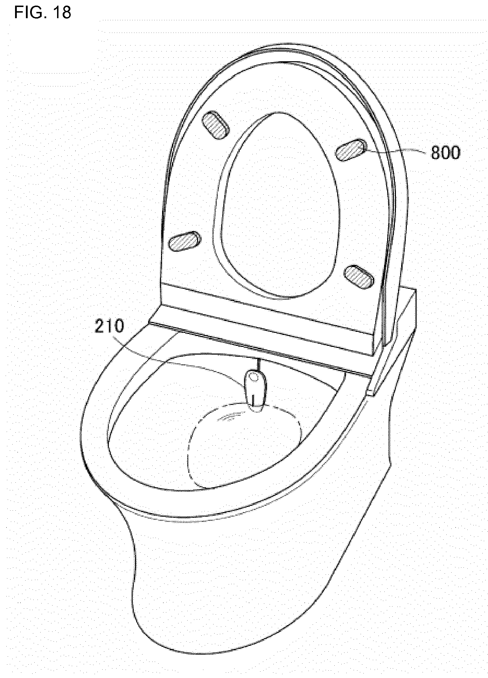

FIG. 18 is a diagram illustrating a configuration example of a toilet in Embodiment 4. As illustrated in FIG. 18, the pressure sensor 800 is attached to a back surface of the toilet seat of the toilet. The pressure sensor 800 measures the weight of the user sitting on the toilet seat. Specifically, the pressure sensor 800 measures a weight applied from the top when the user sits on the toilet seat. Although four pressure sensors 800 are provided on the back surface of the toilet seat in FIG. 18, any number of pressure sensors may be provided. For example, one pressure sensor may be provided.

The pressure sensor 800 is used to calculate a change in weight of the user, and measures the weight applied from top. For example, it is assumed that the pressure sensor 800 measures 30 [Kg] in a state in which the user sits on the toilet seat at a predetermined timing. Thereafter, the pressure sensor 800 measures 31 [kg] in a state in which the user sits on the toilet seat at another timing. In this case, the calculated weight of the user has increased by 1 [kg] between the predetermined timing and the other timing. It should be noted that when a plurality of pressure sensors 800 are included in the toilet seat, the change in weight of the user is calculated on the basis of a sum of measurement values of the plurality of pressure sensors. Thereafter, the calculated amount of change is added to or subtracted from the weight of the user at a predetermined timing, and the weight of the user at another timing is obtained.

It should be noted that the change in weight may be calculated by multiplying the measurement value of the pressure sensor 800 by a predetermined weight according to the number of the pressure sensors 800 attached to the toilet seat, positions thereof, and the like. For example, when there is only one pressure sensor 800 provided on the toilet seat and the toilet seat is supported at four points, the change in weight of the user becomes four times the measurement value. Further, the predetermined weight may be determined in consideration of a position of a centroid of the user, or the like when the user sits on the toilet seat.

Here, the predetermined timing and the other timing may be, for example, a timing when the health monitoring system 500 is used. In this case, it is possible to calculate the change in weight each time the health monitoring system is used, and to calculate the weight of the user each time the health monitoring system is used.

It should be noted that the pressure sensor 800 is capable of measuring an actual weight of the user. In this case, the user places all his weight on the toilet seat. For example, the user lifts his or her foot in a state in which the user sits on the toilet seat so that all the weight is applied to only the toilet seat.

The weight of the user measured using the pressure sensor 800 is stored in the user DB of the storage unit 130 included in the server 100. The pressure sensor 800 stores the weight of the user in the user DB via the measurement unit 210 of the measurement device 200. The user DB updates the weight of the user each time the user is notified of the weight of the user from the measurement device 200.

As described above, in Embodiment 4, it is possible to measure the weight of the user sitting on the toilet seat by attaching the pressure sensor 800 to the toilet seat of the toilet. Therefore, for example, the user can measure and calculate the latest weight of the user each time the toilet is used. Further, since the user can measure and calculate his or her weight just by sitting on the toilet seat, the user can easily measure and calculate the weight as compared with a case in which the weight is measured with a scale.

Further, since the weight is measured and calculated by the pressure sensor 800 attached to the pressure sensor on the toilet seat of the toilet, the latest weight of the user can be measured and calculated each time the user uses the health monitoring system 500. Therefore, it is possible to estimate a disease of the user on the basis of the latest weight of the user at the time of using the health monitoring system.

### (Others)

The health monitoring system according to the present invention can be used as a part of telemedicine in cooperation with a medical institution or the like. For example, each user can store information on related medical institutions, doctors, or the like in a user DB stored in the storage unit 130, and transmit the measurement value and the examination result data in the measurement and examination result DB to the medical institution or the like, for example, at the time of updating the measurement and examination result DB, and a doctor or the like can conduct medical consultation, guidance, or the like regarding health remotely even when a patient is at home on the basis of the transmitted data.

Further, the health monitoring system according to the present invention can also be used for remote medication observation (whether prescribed medicine is taken) or medicine metabolism check (check whether or not prescribed medicine works) of a doctor, a pharmacist, pharmaceutical company, or the like), a service for delivering medicine prescribed according to a state of health or prescription of a doctor from a pharmacy or health check of distant family, or the like. In the health monitoring system according to the present invention, the health monitoring system according to the present invention can also use time series of vital data generated from the measurement and examination result information stored in the storage unit 130 for data marketing business in cooperation with a system of a pharmaceutical company or a health insurance association. Similarly, the vital data can also be used for simulation of how to reduce medical expenses in cooperation with a system of an insurance company or a health insurance association.

Further, the generated vital data can also be used for a service for providing more specific individual health and beauty advice by linking the generated vital data to a daily life log recorded in a wearable device that is in cooperation with the health monitoring system. Further, the vital data can be used for, for example, modeling of what type of health state human beings are living in what state of health by linking the vital data to the life log.

For example, in the case of linking to a life log regarding meals, insufficient nutrients or the like is extracted from the vital data, the extracted nutrients are displayed on the display unit 330 of the user terminal 300, and a meal menu (including food information such as vegetables to be taken) and supplements can be displayed on the display unit 330 of the user terminal 300 and proposed on the basis of the extracted nutrients. Similarly, it is possible to classify types of vital data and propose supplements for supplementing nutrients deficient in a body of a person for each type. A target to which the service is provided is not limited to ordinary households or individuals and can be applied to health management of athletes or the like. Similarly, for cosmetic aspects, personalized cosmetics can be proposed to users who are expected to have trouble, especially, on skin or hair.

Further, the health monitoring system according to the present invention can be used for, for example, modeling what type of genomic human beings live in what state of health, by linking genome interpretation results in addition to vital data and lifelog.

Further, such modeling information can be used as information when an insurance company or the like studies and determines subscription, an insurance fee, or the like on the basis of provided information on a state of health inferred by modeling.

Respective functional units of the server 100, the measurement device 200, and the user terminal 300 may be realized by a logic circuit (hardware) or a dedicated circuit formed in an integrated circuit (an integrated circuit (IC) chip, a large scale integration (LSI), or the like or may be realized by software using a central processing unit (CPU) and a memory. Further, each functional unit may be realized by one or a plurality of integrated circuits, and functions of the plurality of functional units may be realized by one integrated circuit. The LSI is also referred to as a VLSI, a super LSI, an ultra LSI, or the like according to a difference in a degree of integration. It should be noted that, here, a "circuit" may include a meaning of digital processing in a computer, that is, functional processing using software. Further, the circuit may be realized by a reconstructed circuit (for example, FPGA: Field Programmable Gate Away).

When each functional unit of the server 100, the measurement device 200, and the user terminal 300 is realized by software, respective functional units of the server 100, the measurement device 200, or the user terminal 300 include, for example, a CPU that executes instructions of a display information generation program which is software for realizing each function, a read only memory (ROM) or a storage device (these are referred to as a "recording medium") in which the health monitoring program and various pieces of data are recorded to be readable by a computer (or a CPU), and a random access memory (RAM) in which the health monitoring program is developed. The object of the present invention is achieved by the computer (or a CPU) reading the health monitoring program from the recording medium and executing the health monitoring program. As the recording medium, a "non-transitory tangible medium", such as a tape, a disk, a card, a semiconductor memory, or a programmable logic circuit can be used. Further, the health monitoring program may be supplied to the computer via any transmission medium (a communication network, broadcast waves, or the like) capable of transferring the health monitoring program. The present invention can also be realized in a form of a data signal embedded in carrier waves, in which the health monitoring program is embodied through electronic transmission.

It should be noted that the health monitoring program can be implemented by using, for example, script language such as ActionScript, JavaScript (registered trademark), object-oriented programming language such as Objective-C, Java (registered trademark), or markup language such as HTML5.

### EXPLANATION OF REFERENCES

100 Server
110 Communication unit
120 Control unit
130 Storage unit
200 Measurement device
210 Measurement unit (measurement device)
220 User identification unit (measurement device)
230 Control unit (measurement device)
240 Communication unit (measurement device)
250 Storage unit (measurement device)
300 User terminal
310 Communication unit (user terminal)
320 Control unit (user terminal)
330 Display unit (user terminal)
340 Storage unit (user terminal)

## Claims

1. A health monitoring system comprising:
a measurement unit that measures fluid information on fluid in reserved water into which urine of a user of a toilet has flowed;
an interpretation unit that interprets the urine by analyzing a fluid model obtained by modeling a region in which the fluid flows on the basis of the fluid information; and
an inference unit that infers a disease of the user on the basis of urination information of the urine,
wherein the measurement unit includes a cartridge that stores films of which color changes according to components of the reserved water into which the urine has flowed, and extrudes the film to be used for measurement from the cartridge each time the measurement unit measures the fluid information.

2. The health monitoring system according to claim 1, wherein the measurement unit further includes an extrusion mechanism that extrudes the film used for the measurement among a plurality of films stacked and stored in the cartridge to an outside of the cartridge.

3. The health monitoring system according to claim 1 or 2, wherein the measurement unit further includes an arm including a clamping portion that clamps the film extruded from the cartridge, the measurement unit operating the arm to immerse the film clamped by the clamping portion in the reserved water into which the urine of the user has flowed.

4. The health monitoring system according to claim 3,
wherein the measurement unit further includes a rotation mechanism rotatably connected to one end of the arm, and
the arm is rotated by the rotation mechanism to move the clamping portion provided at another end of the arm to a position at which the clamping portion is immersed in the reserved water into which the urine of the user has flowed.

5. The health monitoring system according to claim 3 or 4, further comprising:
an imaging unit including an imaging means that images the film to generate imaging information,
wherein the measurement unit moves the film immersed in the reserved water into which the urine of the user has flowed, to a position at which the imaging unit can image the film by moving the arm.

6. The health monitoring system according to any one of claims 1 to 5,
wherein the cartridge is detachable from the measurement unit, and
in the measurement unit, when the film stored in the cartridge runs out, the cartridge can be replaced with a new cartridge in which a plurality of films are stored.

7. The health monitoring system according to any one of claims 1 to 6, comprising:
a storage unit that stores shape information of a bowl of each toilet and water volume information of the reserved water,
wherein the fluid information includes water temperature information generated by measuring a water temperature of at least one of the reserved water or the reserved water into which the urine has flowed, and
the interpretation unit interprets the urine on the basis of at least one of the shape information and the water volume information, in addition to the fluid information.

8. The health monitoring system according to claim 7,
wherein the measurement unit measures a potential difference between two electrodes immersed in the reserved water or reserved water into which the urine has flowed to generate voltage information, and
the health monitoring system further comprises:
a correction unit that corrects the voltage information on the basis of the water volume information and the urination information including urine volume of the urination; and
an analysis unit that analyzes urine components on the basis of the corrected voltage information.

9. The health monitoring system according to claim 8, wherein when at least one of the water temperature information or the voltage information reaches a predetermined threshold value, the measurement unit starts or ends measurement of at least one of a water temperature and an electric potential difference of the reserved water into which the urine has flowed.

10. The health monitoring system according to any one of claims 1 to 9, wherein the inference unit creates a feature vector from the imaging information, identifies the created feature vector using training data, and infers a disease on the basis of the identified feature vector.

11. The health monitoring system according to any one of claims 1 to 10, further comprising a user identification unit that identifies the user on the basis of user identification information output from a terminal or an IC card possessed by the user,
wherein the inference unit infers a disease of each user on the basis of results of the identification.

12. The health monitoring system according to claim 11, wherein the user identification unit further includes a measurement unit that measures a weight of the user received by a toilet seat of the toilet when the user uses the toilet seat to generate weight information, the user identification unit identifying the user on the basis of the weight information.

13. A health monitoring method comprising:
a measurement step of measuring fluid information on fluid in reserved water into which urine of a user of a toilet has flowed;
an interpretation step of interpreting the urine by analyzing a fluid model obtained by modeling a region in which the fluid flows, on the basis of the fluid information;
an inference step of estimating a disease of the user on the basis of urination information of the urine; and
an extrusion step of extruding a film used for measurement from a cartridge that stores a film of which color changes according to components of reserved water into which the urine has flowed each time the fluid information is measured.

14. A health monitoring program for controlling a computer, the health monitoring program causing the computer to realize:
a measurement function of measuring fluid information on fluid in reserved water into which urine of a user of a toilet has flowed;
an interpretation function of interpreting the urine by analyzing a fluid model obtained by modeling a region in which the fluid flows, on the basis of the fluid information;
an inference function of estimating a disease of the user on the basis of urination information of the urine; and
an extrusion function of extruding a film used for measurement from a cartridge that stores a film of which color changes according to components of reserved water into which the urine has flowed each time the fluid information is measured.
